# EUROPEAN PATENT SPECIFICATION

(11) **EP 4 108 220 B1**
(45) Date of publication and mention of the grant of the patent: **09.04.2025**
(21) Application number: 21181365.4
(22) Date of filing: 24.06.2021
(51) Int. Cl.: A61F 13/514, A61F 13/56, A61F 13/15

(54) **AN ABSORBENT SANITARY ARTICLE AND A METHOD FOR PRODUCING THE SAME**
ABSORBIERENDER HYGIENEARTIKEL UND VERFAHREN ZUR HERSTELLUNG DAVON
ARTICLE SANITAIRE ABSORBANT ET SON PROCÉDÉ DE PRODUCTION

(43) Date of publication of application: 28.12.2022
(73) Proprietor: Fameccanica.Data S.p.A., 66020 San Giovanni Teatino (CH) (IT)
(72) Inventor: GUALTIERI, Diego, I-66020 San Giovanni Teatino (Chieti) (IT); CIPRIANI, Alessandro, I-66020 San Giovanni Teatino (Chieti) (IT)
(74) Representative: Buzzi, Notaro & Antonielli d'Oulx S.p.A.

(56) References cited:
- WO-A1-99/16400
- US-A1- 2011 208 149
- US-A1- 2021 077 307

## Description

### Field of the invention

The present invention relates to absorbent sanitary articles, such as, for example, diapers, training pants, absorbent sanitary products for incontinent adults, etc.

The present invention also relates to a method for producing absorbent sanitary articles.

### Prior art

An absorbent sanitary article typically has a structure that comprises a rectangular-shaped central body or chassis having a front section and a rear section. The chassis is normally formed by a permeable topsheet intended to come into contact with the user's skin when the article is worn, an impermeable backsheet and an absorbent core sandwiched between the topsheet and the backsheet. The front and rear section of the chassis are normally closed around the user's waist by means of hook-and-loop fasteners, better known as Velcro^{©} fastening devices.

In many cases the absorbent sanitary article has a front panel of micro-loop material attached to the front section of the chassis and one pair of elastic back side panels which extend laterally from opposite sides of the rear section of the chassis. The elastic back side panels are provided with fastening tabs, typically comprising micro-hooks pads, which can be releasably attached to the front panel of micro-loop material for closing the absorbent sanitary article around the waist of the user.

The front panel of a hook-and-loop fastener is formed of a fibrous material, for example polypropylene, provided on an upper surface with micro-loops suitable for engaging with the micro-hooks of the side panels. The loop-material is normally fixed to a support typically consisting of a film of plastic material or of a non-woven polypropylene or the like.

EP-A-2540272 describes a multi-layer material for diaper front panels, comprising a support formed of a polyolefinic film and a layer of loop-material formed by a non-woven polyolefinic material. The support and the layer of loop-material are normally secured together by glue.

In conventional solutions, loop material attached to the support is supplied in reels, which are cut transversely to form the front panels which are applied to the outer surface of respective backsheet in the respective front sections. The front panels are normally connected to the backsheet by glue or by ultrasonic welding. Welding is generally not suitable because it can compromise the impermeability of the backsheet.

The backsheet of a diaper is normally a multi-layer composite structure including an inner impermeable film and an outer layer made of a non-woven web connected to the impermeable film by a layer of glue. The front panel is applied on the outer surface of the non-woven web of the backsheet, usually by a layer of glue.

The production of prior art elastic back side panels requires micro-hook pads produced starting from continuous tapes, usually made of relatively rigid thermoplastic material, having continuous or intermittent micro-hook formations integrally formed thereon. The continuous tape with integrally formed micro-hook formations is cut to form a plurality of discrete micro-hook pads. Such discrete micro-hook pads are spaced by a desired pitch and are fixed in spaced positions on a surface of a continuous non-woven support web. The continuous non-woven support web with the micro-hook pads fixed thereon is then cut to form fastening tabs each including a non-woven support web of soft material and a micro-hook pad of relatively rigid thermoplastic material fixed on a surface of the non-woven support web by glue or by welding.

The continuous tapes of micro-hook and micro-loop material are manufactured by suppliers specialized in manufacturing hook-and-loop fasteners. The micro-hook ad micro-loop tapes are packaged in reels which are delivered to the manufacturing plants of absorbent sanitary articles. In the machines for manufacturing absorbent sanitary articles the reels of continuous tapes with integrally formed micro-hook and micro loop formations are unwound, cut, spaced, and fixed to the continuous non-woven support web. All these operations require complex and expensive dedicated equipment which increase the complexity of the machines for manufacturing absorbent sanitary articles. Since the cost of the micro-hook tapes is high, the dimension of the micro-hook pads is kept to the minimum possible, which requires complex equipment for cutting the continuous micro-hook tapes in very small pieces and for feeding such small pieces with high precision to the continuous non-woven support web.

A further drawback of the prior art is that the micro-hook fastening pads of relatively rigid plastic material are positioned on the inner surfaces of the back side panels, which face the skin of the user when the diaper is applied to the user. This may give rise to an unpleasant stiffness and sharpness perception during the application of the sanitary article to the wearer. Also, the user may get scratches or abrasions due to the contact with sharp and rigid micro-hook pads.

WO2010/085492 discloses an apparatus and process for forming micro-hooks on a substrate for use as hook-type fasteners in touch fastening systems, wherein vibration energy is used to soften a substrate which is positioned between a mold and a source of vibration. The mold includes a plurality of cavities into which the softened substrate is forced to form the projections. The substrate may comprise a film, sheet, web, composite, laminate, etc. The source of vibration may be an ultrasonic horn. The process to form such micro-hooks may be operated in a continuous, semi-continuous or intermittent manner.

WO99/16400 A1, US2021/0077307 A1 and US2011/0208149 A1 disclose absorbent articles with hook-and-loop fastening means.

### Object and summary of the invention

The object of the present invention is to provide an absorbent sanitary article which overcomes the problems of the prior art.

According to the invention, this object is achieved by an absorbent sanitary article having the features of claim 1.

According to another aspect, the invention relates to a method for manufacturing absorbent sanitary articles having the features of claim 7.

The claims form an integral part of the technical disclosure provided here in relation to the invention.

### Brief description of the drawings

The present invention will now be described with reference to the attached drawings, provided purely by way of non-limiting example, wherein:
- Figure 1 is a perspective view of an absorbent sanitary article according to the present invention,
- Figures 2-5 are schematic plan views showing different embodiments of absorbent sanitary articles according to the present invention,
- Figure 6 and 7 are schematic perspective views showing a method for manufacturing the backsheet of the absorbent sanitary articles according to the present invention,
- Figure 8 is a schematic plan view taken along the arrow VIII of figure 6,
- Figure 9 is a schematic cross-section taken along the line IX-IX of figure 8, and
- Figure 10 is a schematic cross-section taken along the line X-X of figure 2.

It should be appreciated that the attached drawings are schematic and not to scale with respect to real products. Various figures may not be represented in the same scale. Also, in various figures some elements may not be shown to better show other elements.

### Detailed description

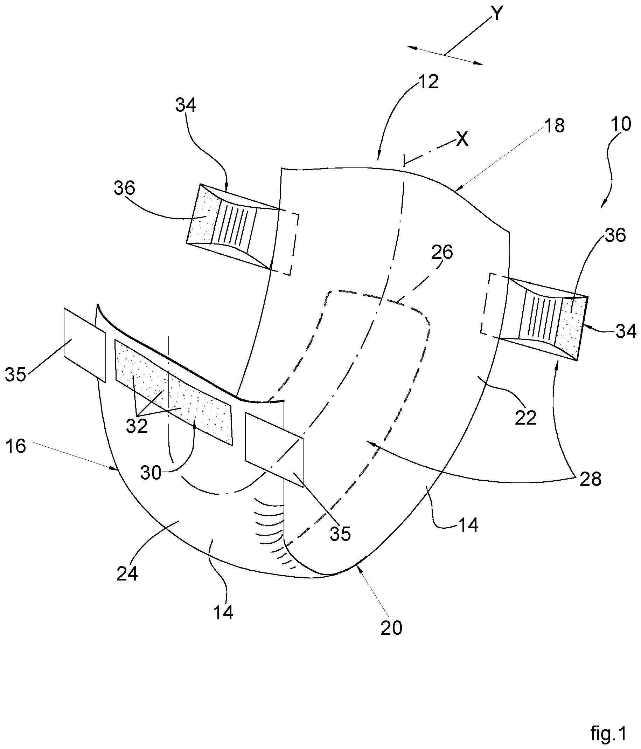

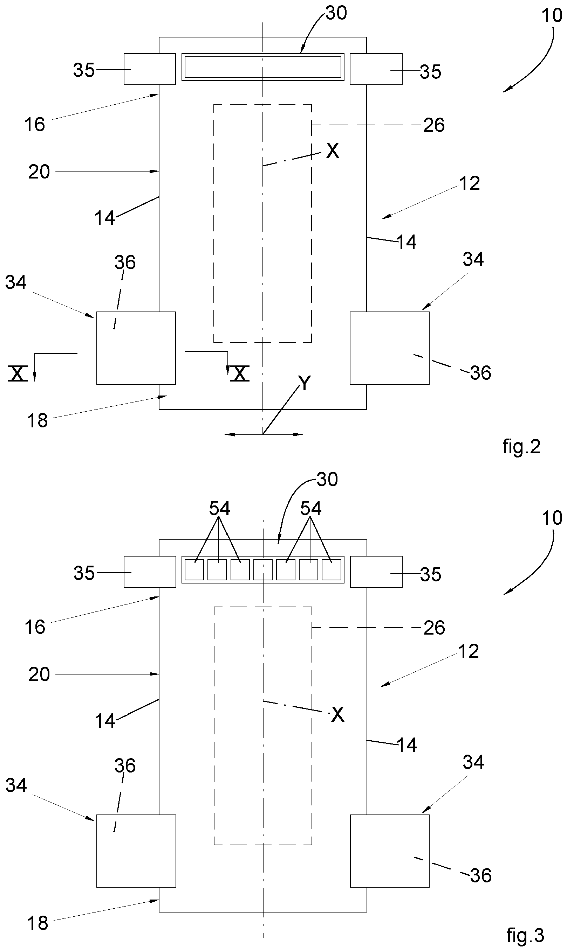

With reference to Figures 1-5, numeral 10 indicates an absorbent sanitary article according to the present invention. Figure 1 shows the absorbent sanitary article 10 in the configuration in which it is worn. Figures 2-5 show various embodiments of absorbent sanitary articles 10 according to the present invention in a flat configuration.

The absorbent sanitary article 10 comprises a chassis 12 elongated along a longitudinal axis X. The chassis 12 has two side edges 14 parallel to the longitudinal axis X, a front section 16 and a rear section 18. The front section 16 and the rear section 18 in use are closed around the user's waist. Between the front section 16 and the rear section 18 a groin section 20 extends which, in use, is arranged between the legs of the user.

The chassis 12 comprises a topsheet 22 made of a permeable material which, in use, is in contact with the user's skin, an impermeable backsheet 24 and an absorbent core 26 sandwiched between the topsheet 22 and the backsheet 24. The chassis 12 may comprise additional components as usual in the field of absorbent sanitary articles, such as elastic leg cuffs, acquisition and diffusion layers, etc.

The absorbent sanitary article 10 comprises a hook-and-loop fastening device 28 configured for fastening the front section 16 and the rear section 18 to each other.

The hook-and-loop fastening device 28 comprise a micro-hook front panel 30 located in the front section 16 of the chassis 12. The micro-hook front panel 30 includes a plurality of micro-hooks 32 integrally formed on a portion of the outer surface of the backsheet 24, which are formed as it will be disclosed in the following.

The hook-and-loop fastening device 28 comprise two back side panels 34 attached to the rear section 18 of the chassis 12 and extending laterally beyond respective side edges 14 in a transverse direction Y orthogonal to the longitudinal axis X. The side panels 34 have respective micro-loop areas 36 configured for forming a releasable surface connection with the micro-hook front panel 30. The micro-loop areas 36 may be formed by a portion of a non-woven web. The side panels 34 may be elastically stretchable the transverse direction Y.

The absorbent sanitary article 10 may comprises a pair of front side panels 35 projecting laterally from opposite sides of the front section 16, having the function of facilitating attachment of the back side panels 34 to the micro-hook front panel 30 when the absorbent sanitary article 10 is closed around the user's waist.

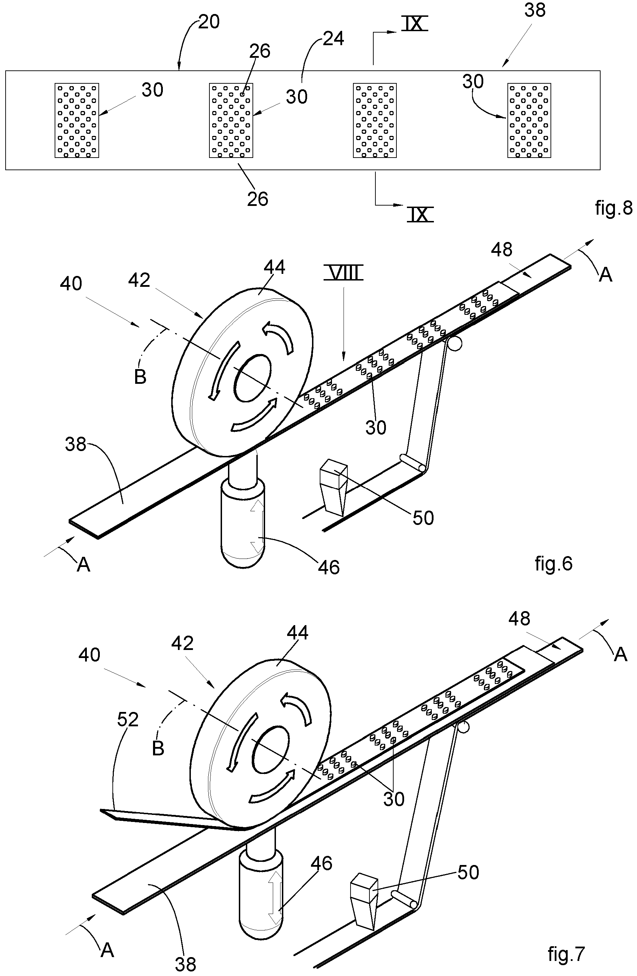

The backsheet 24 with the integral micro-hook front panel 30 may be produced by a method disclosed in WO2010/085492 and schematically shown in figures 6 and 7.

With reference to Figure 6, a continuous non-woven web 38 moving in its longitudinal direction A passes through a micro-hook forming unit 40. The micro-hook forming unit 40 comprises a moulding roller 42 rotating about an axis B transversal to the longitudinal direction A of the continuous non-woven web 38. The moulding roller 42 has a plurality of micro-cavities open on its outer cylindrical surface 44. The micro-hook forming unit 40 comprises an ultrasonic horn 46 which compresses the continuous non-woven web 38 against the outer cylindrical surface 44 of the moulding roller 42 as the continuous non-woven web 38 moves in the direction A.

The vibrating energy produced by the ultrasonic horn 46 liquifies or fluidifies locally the non-woven material of the continuous non-woven web 38, which penetrates in a fluid or liquid state in the micro-cavities of the moulding roller 42. The material of the non-woven web 38 which penetrates in the cavities of the molding roller 29 cools in contact with the walls of the micro-cavities and forms micro-hook front panels 30 integral with the continuous non-woven web 38.

As shown in figure 8, at the exit of the micro-hook forming unit 40 the continuous non-woven web 38 has an array of micro-hook front panels 30 spaced apart from each other in the longitudinal direction A.

The array of spaced apart micro-hook front panels 30 may be obtained by providing on the outer surface 44 of the moulding roller 42 an intermittent pattern of micro-cavities. Alternatively, the moulding roller 42 may have a continuous pattern of micro-cavities and the array of spaced apart micro-hook front panels 30 may be formed by intermittently turning off the ultrasonic horn 46. As a further alternative, the moulding roller 42 may have a continuous pattern of micro-cavities and the array of spaced apart micro-hook front panels 30 may be formed by intermittently detaching the ultrasonic horn 46 from the outer cylindrical surface 44 of the moulding roller 42.

At the exit of the micro-hook forming unit 40 the continuous non-woven web 38 with the array of spaced apart integrally formed micro-hook front panels 30 is overlapped and fixed to a continuous impermeable film 48 moving in the same direction A as the continuous non-woven web 38. With reference to figures 6, 7 and 9, the continuous non-woven web 38 and the continuous impermeable film 48 may be fixed to each other by a layer of glue 49 which may be applied on a surface of the continuous impermeable film 48 by a glue dispenser 50.

The continuous non-woven web 38 and the continuous impermeable film 48 form a continuous backsheet comprising an inner impermeable film 48 and an outer non-woven layer 38 fixed to the inner impermeable film 48 e.g. by a layer of glue 49, wherein an array of micro-hook front panels 30 is integrally formed on an outer surface of the outer non-woven layer 38.

In order to form micro-hooks with dimensions sufficient for an effective hook-and-loop connection, the continuous non-woven layer 38 should have a weight of 20-120 g/m².

With reference to figure 7, in a possible embodiment, an additional continuous web 52 may be applied on the continuous non-woven web 38 before passing the two webs 38, 52 through the micro-hook forming unit 42. The additional continuous web 52 may be a thermoplastic film or a non-woven web of a material different or identical to the material of the continuous non-woven web 38. After passing through the micro-hook forming unit 40, the additional continuous web 52 and the continuous non-woven web 38 form a unitary layer with integrally formed micro-hook front panels 30. The use of an additional web 52 may allow a reduction of the weight of the continuous non-woven web 38. For instance, in a possible embodiment two continuous non-woven webs 38, 52 may be used, each having a weight of 40 g/m2, which is a standard weight of non-woven webs in the field of absorbent sanitary articles.

In a possible embodiment, the micro-hook forming unit 40 may be a thermomechanical forming unit, in which the material of the non-woven web 38, or webs 38, 52, is locally liquified or fluidified by heating and compression.

With reference to figure 3, in a possible embodiment the micro-hooks 32 of the micro-hook front panel 30 may be arranged in a plurality of discrete pads 54.

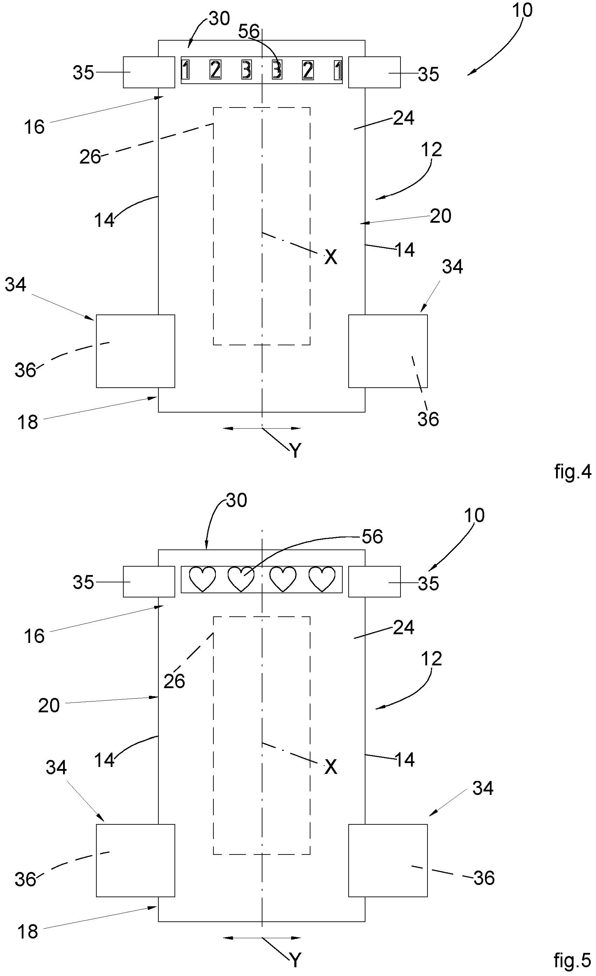

With reference to figures 4 and 5, in a possible embodiment the micro-hooks 32 of the micro-hook front panel 30 may be arranged in a pattern 56 forming images, writings or numbers. The numbers may provide a visual indication of the position for attaching the back side panels 34. Images and writings may indicate the trademark of the producer or the trade-name of the article.

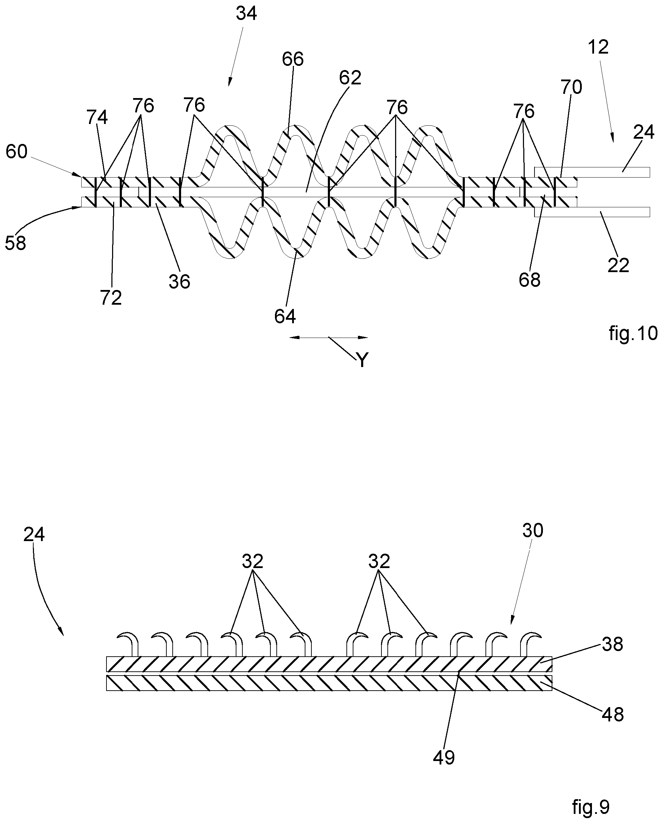

With reference to figure 10, the back side panel 34 comprises a first non-woven web 58, a second non-woven web 60 and an elastic film 62 sandwiched between the first and second non-woven web 58, 60. The elastic film 18 is elastically stretchable in a transversal direction Y.

The first and second non-woven web 58, 60 have respective pleated central portions 64, 66, respective non-pleated proximal portions 68, 70 and respective non-pleated distal portions 72, 74. The first and second non-woven web 58, 60 and the elastic film 62 are fixed to each other, e.g. by a pattern of spot welds 76. The non-pleated proximal portions 68, 70 are fixed to the chassis 12. The surface of the non-pleated distal portion 72 forms a micro-loop area 36 which forms the loop portion of a hook-and-loop fastening connection.

The back side panels 34 have a soft feeling to the touch because they do not have micro-hooks. The back side panels 34 do not require the application of discrete micro-hook pads and this involves considerable advantages in that there is no need for complex and expensive devices for applying discrete elements at the distal portions of the side panels 34.

The micro-hook front panel 30 is relatively soft as compared to the prior art micro-hook pads, because the micro-hooks 32 are obtained starting from non-woven material, which is softer than the thermoplastic material forming the traditional micro-hooks of hook-and-loop fastening devices. The micro-hook front panel 30 is located on an outer surface of the absorbent sanitary article, which does not contact the skin of the user when the absorbent sanitary article is applied to the user.

The hook-and-loop fastening device of the absorbent sanitary article according to the present invention is integrally formed on materials which are already present in the structure of the absorbent sanitary article. The hook-and-loop fastening device 28 of the absorbent sanitary article according to the present invention does not require reels of micro-hook ad micro-loop tapes to be transported and delivered to the manufacturing plants of absorbent sanitary articles. The invention has therefore considerable advantages regarding lower costs of materials, simpler manufacturing machines and an improved sustainability.

The absorbent sanitary articles 10 according to the present invention may be manufactured by a method comprising:
- integrally forming an array of micro-hook front panels 30 spaced apart from each other on an outer surface of a continuous backsheet 24,
- sandwiching absorbent cores 26 between a continuous topsheet 22 and the continuous backsheet 24 with integrally formed micro-hook front panels 30, so as to form a chain of chassis 12, wherein the micro-hook front panels 30 are positioned in respective front sections 16 of respective chassis 12, and
- providing said chain of chassis 12 with an array of pair of back side panels 34 attached to respective rear sections 18 of respective chassis 12 and extending laterally beyond respective side edges 14 in a transverse direction (Y) orthogonal to said longitudinal axis (X), wherein the back side panels 34 have respective micro-loop areas 36 configured for forming a hook-and-loop connection with the micro-hook 35 front panels 30. The back side panels 34 are elastically stretchable in said transverse direction (Y). Each of said back side panels comprises:
   - a first and a second non-woven web 58, 60, having respective pleated central portions 64, 66 and respective non-pleated proximal and distal portions 68, 70, 72, 74 on opposite sides of said pleated central portions 64, 66, and
   - an elastic film 62 sandwiched between said pleated central portions 64, 66 of said first and a second non-woven web 58, 60.
The outer surface of the distal portion 72 of said first non-woven web 58 forms said micro-loop area 36.

The chain of chassis 12 is then cut transversely to form individual absorbent sanitary articles 10.

The formation of the micro-hook front panels 30 on the outer surface of the continuous backsheet 24 may comprise:
- providing an inner impermeable film 48 and an outer non-woven layer 38, 52,
- integrally forming the micro-hook front panels 30 on portions of the outer surface of the outer non-woven layer 38, 52, and
- fixing the outer non-woven layer 38, 52 with integrally formed micro-hook front panels 30 to the inner impermeable film 48.

Of course, without prejudice to the principle of the invention, the details of construction and the embodiments can be varied, even significantly, with respect to those illustrated here without departing from the scope of the invention as defined by the following claims.

## Claims

1. An absorbent sanitary article comprising:
- a chassis (12) having a longitudinal axis (X), two side edges (14), a front section (16) and a rear section (18), the chassis including a topsheet (22), a backsheet (24) having an outer surface, and an absorbent core (26) sandwiched between the topsheet (22) and the backsheet (24),
- a micro-hook front panel (30) including a plurality of micro-hooks (32) integrally formed on a portion of the outer surface of said backsheet (24) in said front section (16) of the chassis (12), and
- two back side panels (34) attached to said rear section (18) of the chassis (12) and extending laterally beyond respective side edges (14) in a transverse direction (Y) orthogonal to said longitudinal axis (X), the back side panels (34) having respective micro-loop areas (36) configured for forming a hook-and-loop connection with said micro-hook front panel (30), wherein said back side panels (34) are elastically stretchable in said transverse direction (Y), and wherein each of said back side panels comprises:
- a first and a second non-woven web (58, 60), having respective pleated central portions (64, 66) and respective non-pleated proximal and distal portions (68, 70, 72, 74) on opposite sides of said pleated central portions (64, 66), and
- an elastic film (62) sandwiched between said pleated central portions (64, 66) of said first and a second non-woven web (58, 60),
wherein the outer surface of the distal portion (72) of said first non-woven web (58) forms said micro-loop area (36).

2. The absorbent sanitary article of claim 1, wherein said backsheet (24) comprises an inner impermeable film (48) and an outer non-woven layer (38, 52) fixed to the inner impermeable film (48), wherein said micro-hook front panel (30) is integrally formed on said outer non-woven layer (38, 52).

3. The absorbent sanitary article of claim 2, wherein said outer non-woven layer (38, 52) has a weight of 20-120 g/m².

4. The absorbent sanitary article of claim 2 or claim 3, wherein said outer non-woven layer comprises two overlapped non-woven webs (38, 52).

5. The absorbent sanitary article of any of the preceding claims, wherein the micro-hooks (32) of said micro-hook front panel (30) are arranged in a pattern (56) forming images, and/or writings and/or numbers.

6. The absorbent sanitary article of any of the preceding claims, wherein the micro-hooks (32) of the micro-hook front panel (30) are arranged in a plurality of discrete pads (54).

7. A method for producing an absorbent sanitary article according to claim 1, comprising:
- integrally forming an array of micro-hook front panels (30) spaced apart from each other on an outer surface of a continuous backsheet (24),
- sandwiching absorbent cores (26) between a continuous topsheet (22) and the continuous backsheet (24) with integrally formed micro-hook front panels (30), so as to form a chain of chassis (12), wherein the micro-hook front panels (30) are positioned in respective front sections (16) of the chain of chassis (12),
- providing said chain of chassis (12) with an array of pair of back side panels (34) attached to respective rear sections (18) of the chain of chassis (12) and extending laterally beyond respective side edges (14) in a transverse direction (Y), wherein the back side panels (34) have respective micro-loop areas (36) configured for forming a hook-and-loop connection with the micro-hook front panels (30), wherein said back side panels (34) are elastically stretchable in said transverse direction (Y), and wherein each of said back side panels comprises:
- a first and a second non-woven web (58, 60), having respective pleated central portions (64, 66) and respective non-pleated proximal and distal portions (68, 70, 72, 74) on opposite sides of said pleated central portions (64, 66), and
- an elastic film (62) sandwiched between said pleated central portions (64, 66) of said first and a second non-woven web (58, 60),
wherein the outer surface of the distal portion (72) of said first non-woven web (58) forms said micro-loop area (36), and
- transversely cutting the chain of chassis (12) to form individual absorbent sanitary articles (10).

8. The method of claim 7, wherein integrally forming said micro-hook front panels (30) on the outer surface of the continuous backsheet (24) comprises:
- providing an inner impermeable film (48) and an outer non-woven layer (38, 52),
- integrally forming said array of micro-hook front panels (30) on portions of an outer surface of said outer non-woven layer (38, 52), and
- fixing said outer non-woven layer (38, 52) with integrally formed micro-hook front panels (30) to the inner impermeable film (48).

9. The method of claim 8, wherein said outer non-woven layer (38, 52) has a weight of 20-120 g/m².

10. The method of claim 8 or claim 9, comprising forming said outer non-woven layer by overlapping two non-woven webs (38, 52).

11. The method of any of claims 7-10, comprising arranging said micro-hooks (32) of each of said micro-hook front panels (30) in a pattern (56) forming images, and/or writings and/or numbers and/or a plurality of discrete pads (54).

12. The method of any of claims 7-11, wherein said micro-hooks (32) of said micro-hook front panels (30) are integrally formed in a non-woven layer (38, 52) by locally liquifying or fluidifying the non-woven layer (38, 52) and inserting the liquified or fluidified non-woven material into cavities of a molding roller (42).

13. The method of claim 12, wherein locally liquifying or fluidifying the non-woven material is obtained by subjecting non-woven layer (38, 52) to ultrasonic compression or to thermomechanical compression.

## Patentansprüche

1. Absorbierender Hygieneartikel, umfassend:
- ein Gehäuse (12), das eine Längsachse (X), zwei Seitenkanten (14), einen vorderen Abschnitt (16) und einen hinteren Abschnitt (18) aufweist, wobei das Gehäuse eine Oberschicht (22), eine Unterschicht (24), die eine äußere Oberfläche aufweist, und einen zwischen der Oberschicht (22) und der Unterschicht (24) angeordneten absorbierenden Kern (26) einschließt,
- eine Mikrohaken-Frontplatte (30), die eine Vielzahl von Mikrohaken (32) einschließt, die an einem Abschnitt der äußeren Oberfläche der Unterschicht (24) im vorderen Abschnitt (16) des Gehäuses (12) integral ausgebildet sind, und
- zwei Rückseitenplatten (34), die an dem hinteren Abschnitt (18) des Gehäuses (12) angebracht sind und sich seitlich über die jeweiligen Seitenkanten (14) in einer Querrichtung (Y) erstrecken, die orthogonal zur Längsachse (X) ist, wobei die Rückseitenplatten (34) jeweilige Mikroösenbereiche (36) aufweisen, die zum Bilden einer Haken- und Ösenverbindung mit der Mikrohaken-Frontplatte (30) konfiguriert sind, wobei die Rückseitenplatten (34) in der Querrichtung (Y) elastisch dehnbar sind, und wobei jedes der Rückseitenplatten umfasst:
- eine erste und eine zweite Vliesbahn (58, 60), die jeweilige gefaltete Mittelabschnitte (64, 66) und jeweilige nicht gefaltete proximale und distale Abschnitte (68, 70, 72, 74) auf gegenüberliegenden Seiten der gefalteten Mittelabschnitte (64, 66) aufweisen, und
- eine elastische Folie (62), die zwischen den gefalteten Mittelabschnitten (64, 66) der ersten und einer zweiten Vliesbahn (58, 60) angeordnet ist,
- wobei die äußere Oberfläche des distalen Abschnitts (72) der ersten Vliesbahn (58) den Mikroösenbereich (36) bildet.

2. Absorbierender Hygieneartikel nach Anspruch 1, wobei die Unterschicht (24) eine innere undurchlässige Folie (48) und eine äußere, an der inneren undurchlässigen Folie (48) befestigte Vliesschicht (38, 52) umfasst, wobei die Mikrohaken-Frontplatte (30) auf der äußeren Vliesschicht (38, 52) integral ausgebildet ist.

3. Absorbierender Hygieneartikel nach Anspruch 2, wobei die äußere Vliesschicht (38, 52) ein Gewicht von 20-120 g/m² aufweist.

4. Absorbierender Hygieneartikel nach Anspruch 2 oder Anspruch 3, wobei die äußere Vliesschicht zwei überlappende Vliesbahnen (38, 52) umfasst.

5. Absorbierender Hygieneartikel nach einem der vorstehenden Ansprüche, wobei die Mikrohaken (32) der Mikrohaken-Frontplatte (30) in einem Muster (56) angeordnet sind, das Bilder und/oder Schriftzüge und/oder Zahlen bildet.

6. Absorbierender Hygieneartikel nach einem der vorstehenden Ansprüche, wobei die Mikrohaken (32) der Mikrohaken-Frontplatte (30) in einer Vielzahl diskreter Pads (54) angeordnet sind.

7. Verfahren zum Herstellen eines absorbierenden Hygieneartikels nach Anspruch 1, umfassend:
- integrales Bilden einer Anordnung von Mikrohaken-Frontplatten (30), die auf einer äußeren Oberfläche einer kontinuierlichen Unterschicht (24) voneinander beabstandet sind,
- Einschließen von absorbierenden Kernen (26) zwischen einer kontinuierlichen Oberschicht (22) und der kontinuierlichen Unterschicht (24) mit integral ausgebildeten Mikrohaken-Frontplatten (30), um eine Kette von Gehäusen (12) zu bilden, wobei die Mikrohaken-Frontplatten (30) in jeweiligen Frontabschnitten (16) der Gehäusekette (12) positioniert sind,
- Bereitstellen der Gehäusekette (12) mit einer Anordnung von Paaren von Rückseitenplatten (34), die an jeweiligen hinteren Abschnitten (18) der Gehäusekette (12) angebracht sind und sich seitlich über jeweilige Seitenkanten (14) in einer Querrichtung (Y) hinaus erstrecken, wobei die Rückseitenplatten (34) jeweilige Mikroösenbereiche (36) aufweisen, die zum Bilden einer Haken- und Ösenverbindung mit den Mikrohaken-Frontplatten (30) konfiguriert sind, wobei die Rückseitenplatten (34) in der Querrichtung (Y) elastisch dehnbar sind, und wobei jedes der Rückseitenplatten umfasst:
- eine erste und eine zweite Vliesbahn (58, 60), die jeweilige gefaltete Mittelabschnitte (64, 66) und jeweilige nicht gefaltete proximale und distale Abschnitte (68, 70, 72, 74) auf gegenüberliegenden Seiten der gefalteten Mittelabschnitte (64, 66) aufweisen, und
- eine elastische Folie (62), die zwischen den gefalteten Mittelabschnitten (64, 66) der ersten und einer zweiten Vliesbahn (58, 60) angeordnet ist,
wobei die äußere Oberfläche des distalen Abschnitts (72) der ersten Vliesbahn (58) den Mikroösenbereich (36) bildet, und Querschneiden der Gehäusekette (12), um einzelne absorbierende Hygieneartikel (10) zu bilden.

8. Verfahren nach Anspruch 7, wobei das integrale Bilden der Mikrohaken-Frontplatten (30) auf der äußeren Oberfläche der kontinuierlichen Unterschicht (24) umfasst:
- Bereitstellen einer inneren undurchlässigen Folie (48) und einer äußeren Vliesschicht (38, 52),
- Integrales Bilden der Anordnung von Mikrohaken-Frontplatten (30) auf Abschnitten einer äußeren Oberfläche der äußeren Vliesschicht (38, 52), und
- Befestigen der äußeren Vliesschicht (38, 52) mit integral gebildeten Mikrohaken-Frontplatten (30) an der inneren undurchlässigen Folie (48).

9. Verfahren nach Anspruch 8, wobei die äußere Vliesschicht (38, 52) ein Gewicht von 20-120 g/m² aufweist.

10. Verfahren nach Anspruch 8 oder Anspruch 9, umfassend ein Bilden der äußeren Vliesschicht durch Überlappen zweier Vliesbahnen (38, 52).

11. Verfahren nach einem der Ansprüche 7-10, umfassend ein Anordnen der Mikrohaken (32) jeder der Mikrohaken-Frontplatten (30) in einem Muster (56), das Bilder und/oder Schriftzüge und/oder Zahlen und/oder eine Vielzahl diskreter Pads (54) bildet.

12. Verfahren nach einem der Ansprüche 7-11, wobei die Mikrohaken (32) der Mikrohaken-Frontplatten (30) in einer Vliesschicht (38, 52) integral gebildet sind, indem die Vliesschicht (38, 52) lokal verflüssigt oder fluidisiert wird und das verflüssigte oder fluidisierte Vliesmaterial in Hohlräume einer Formwalze (42) eingefügt wird.

13. Verfahren nach Anspruch 12, wobei das lokale Verflüssigen oder Fluidisieren des Vliesmaterials dadurch erreicht wird, dass die Vliesschicht (38, 52) einer Ultraschallkompression oder einer thermomechanischen Kompression unterzogen wird.

## Revendications

1. Article sanitaire absorbant comprenant :
- un châssis (12) présentant un axe longitudinal (X), deux bords latéraux (14), une section avant (16) et une section arrière (18), le châssis incluant une feuille supérieure (22), une feuille arrière (24) présentant une surface externe, et un coeur absorbant (26) pris en sandwich entre la feuille supérieure (22) et la feuille arrière (24),
- un panneau avant (30) à micro-crochets incluant une pluralité de micro-crochets (32) formés d'un seul tenant sur une portion de la surface externe de ladite feuille arrière (24) dans ladite section avant (16) du châssis (12), et
- deux panneaux latéraux arrière (34) fixés à ladite section arrière (18) du châssis (12) et s'étendant latéralement au-delà des bords latéraux (14) respectifs dans une direction transversale (Y) orthogonale audit axe longitudinal (X), les panneaux latéraux arrière (34) présentant des zones à micro-boucles (36) respectives configurées pour former une connexion auto-agrippante avec ledit panneau avant (30) à micro-crochets, dans lequel lesdits panneaux latéraux arrière (34) sont élastiquement étirables dans ladite direction transversale (Y), et dans lequel chacun desdits panneaux latéraux arrière comprend :
- une première et une seconde bande non tissée (58, 60), présentant des portions centrales plissées (64, 66) respectives et des portions proximales et distales non plissées (68, 70, 72, 74) respectives sur des côtés opposés desdites portions centrales plissées (64, 66), et
- un film élastique (62) pris en sandwich entre lesdites portions centrales plissées (64, 66) de ladite première et de ladite seconde bande non tissée (58, 60),
- dans lequel la surface externe de la portion distale (72) de ladite première bande non tissée (58) forme ladite zone à micro-boucles (36).

2. Article sanitaire absorbant selon la revendication 1, dans lequel ladite feuille arrière (24) comprend un film imperméable interne (48) et une couche non tissée (38, 52) externe fixée au film imperméable interne (48), dans lequel ledit panneau avant (30) à micro-crochets est formé d'un seul tenant sur ladite couche non tissée (38, 52) externe.

3. Article sanitaire absorbant selon la revendication 2, dans lequel ladite couche non tissée (38, 52) externe présente un poids de 20 à 120 g/m².

4. Article sanitaire absorbant selon la revendication 2 ou la revendication 3, dans lequel ladite couche non tissée externe comprend deux bandes non tissées (38, 52) en chevauchement.

5. Article sanitaire absorbant selon l'une quelconque des revendications précédentes, dans lequel les micro-crochets (32) dudit panneau avant (30) à micro-crochets sont agencés en un motif (56) formant des images, et/ou des écritures et/ou des nombres.

6. Article sanitaire absorbant selon l'une quelconque des revendications précédentes, dans lequel les micro-crochets (32) du panneau avant (30) à micro-crochets sont agencés en une pluralité de blocs (54) discrets.

7. Procédé de production d'un article sanitaire absorbant selon la revendication 1, comprenant :
- la formation d'un seul tenant d'un réseau de panneaux avant (30) à micro-crochets espacés les uns des autres sur une surface externe d'une feuille arrière (24) continue,
- la prise en sandwich de coeurs absorbants (26) entre une feuille supérieure (22) continue et la feuille arrière (24) continue avec des panneaux avant (30) à micro-crochets formés d'un seul tenant, de façon à former une chaîne de châssis (12), dans lequel les panneaux avant (30) à micro-crochets sont positionnés dans des sections avant (16) respectives de la chaîne de châssis (12),
- le fait de munir ladite chaîne de châssis (12) d'un réseau de paire de panneaux latéraux arrière (34) fixés à des sections arrière (18) respectives de la chaîne de châssis (12) et s'étendant latéralement au-delà de bords latéraux (14) respectifs dans une direction transversale (Y), dans lequel
les panneaux latéraux arrière (34) présentent des zones à micro-boucles (36) respectives configurées pour former une connexion auto-agrippante avec les panneaux avant (30) à micro-crochets, dans lequel lesdits panneaux latéraux arrière (34) sont élastiquement étirables dans ladite direction transversale (Y), et dans lequel chacun desdits panneaux latéraux arrière comprend :
- une première et une seconde bande non tissée (58, 60), présentant des portions centrales plissées (64, 66) respectives et des portions proximales et distales non plissées (68, 70, 72, 74) respectives sur des côtés opposés desdites portions centrales plissées (64, 66), et
- un film élastique (62) pris en sandwich entre lesdites portions centrales plissées (64, 66) de ladite première et de ladite seconde bande non tissée (58, 60),
dans lequel la surface externe de la portion distale (72) de ladite première bande non tissée (58) forme ladite zone à micro-boucles (36), et
la découpe transversale de la chaîne de châssis (12) pour former des articles sanitaires absorbants (10) individuels.

8. Procédé selon la revendication 7, dans lequel la formation d'un seul tenant desdits panneaux avant (30) à micro-crochets sur la surface externe de la feuille arrière (24) continue comprend :
- la fourniture d'un film imperméable interne (48) et d'une couche non tissée (38, 52) externe,
- la formation d'un seul tenant dudit réseau de panneaux avant (30) à micro-crochets sur des portions d'une surface externe de ladite couche non tissée (38, 52) externe, et
- la fixation de ladite couche non tissée (38, 52) externe avec des panneaux avant (30) à micro-crochets formés d'un seul tenant au film imperméable interne (48).

9. Procédé selon la revendication 8, dans lequel ladite couche non tissée externe (38, 52) présente un poids de 20 à 120 g/m².

10. Procédé selon la revendication 8 ou la revendication 9, comprenant la formation de ladite couche non tissée externe par chevauchement de deux bandes non tissées (38, 52).

11. Procédé selon l'une quelconque des revendications 7 à 10, comprenant l'agencement desdits micro-crochets (32) de chacun desdits panneaux avant (30) à micro-crochets en un motif (56) formant des images, et/ou des écritures et/ou des nombres et/ou une pluralité de blocs (54) discrets.

12. Procédé selon l'une quelconque des revendications 7 à 11, dans lequel lesdits micro-crochets (32) desdits panneaux avant (30) à micro-crochets sont formés d'un seul tenant en une couche non tissée (38, 52) en liquéfiant ou en fluidifiant localement la couche non tissée (38, 52) et en insérant le matériau non tissé liquéfié ou fluidifié dans des cavités d'un rouleau de moulage (42).

13. Procédé selon la revendication 12, dans lequel la liquéfaction ou la fluidification locale du matériau non tissé est obtenue en soumettant une couche non tissée (38, 52) à une compression ultrasonore ou à une compression thermomécanique.
